# EUROPEAN PATENT APPLICATION

(11) **EP 4 603 575 A1**
(43) Date of publication of application: **20.08.2025**
(21) Application number: 23890732.3
(22) Date of filing: 13.11.2023
(51) Int. Cl.: C12N 1/20, C12P 17/18, C12R 1/01

(54) **HYPHOMICROBIUM DENITRIFICNS AND METHOD FOR FERMENTING SAME TO PREPARE PYRROLOQUINOLINE QUINONE**

(30) Priority: 14.11.2022 CN 202211417029
(71) Applicant: Hubei Magic Health Technology Co., Ltd., Hubei 443000 (CN)
(72) Inventor: CHEN, Fuchen, Yichang, Hubei 443007 (CN); ZHANG, Heng, Yichang, Hubei 443007 (CN); YE, Shihao, Yichang, Hubei 443007 (CN); CHEN, Yumin, Yichang, Hubei 443007 (CN); ZOU, Le, Yichang, Hubei 443007 (CN)
(74) Representative: HGF
(86) International application number: PCT/CN2023/131340
(87) International publication number: WO 2024/104299

(57) **Abstract**

Provided is *Hyphomicrobium denitrificans* MQ004-2, which was deposited at the China Center for Type Culture Collection on July 8, 2022, with an accession number of CCTCC NO: M20221066. The problems that a PQQ fermentation yield is not high, and the metabolic process of Hyphomicrobium denitrificans is unstable, which consequently makes it difficult to achieve large-scale production are solved. The strain has a high titer level, a stable titer level between batches, a small methanol consumption difference, and good strain metabolism stability and facilitates large-scale production.

## Description

### TECHNICAL FIELD

The present invention relates to the technical field of microorganisms, and in particular to a strain of *Hyphomicrobium denitrificns* and a method for fermenting same to prepare pyrroloquinoline quinone.

### BACKGROUND

Pyrroloquinoline quinone (PQQ) is a novel organic redox coenzyme distinct from nicotinamide nucleotides (NAD⁺ and NADP⁺) and flavin nucleotides (FAD and FMN). To date, the capability of PQQ in catalyzing oxidation-reduction reactions is far stronger than known bioactive molecules. The current synthetic methods of PQQ include chemical synthesis and biological synthesis, and the latter has the characteristics of being natural, being environmentally friendly, low cost, fewer steps in synthesis, easier separation, and no toxic and harmful impurities in the finished products. To date, the ability to synthesize PQQ is only found in microorganisms and is widely present in gram-negative bacteria. Methylotrophic bacteria are gram-negative bacteria taking one-carbon methyl compounds (except methane) such as methanol as the sole carbon source, in which methanol needs to be oxidized into formaldehyde by methanol dehydrogenase and subsequently assimilated into methylene tetrahydrofolic acid to enter the serine cycle. Therefore, methylotrophic bacteria have to synthesize PQQ to activate the activity of methanol dehydrogenase to meet their normal physiological and metabolic requirements. The main reason for the synthesis of a large amount of PQQ during the growth of methylotrophic bacteria is that one-carbon compounds such as methanol, methylamine, and the like are the sole carbon source and energy source for growth, and PQQ, the co-factor of one-carbon compound dehydrogenases, has to be induced and synthesized in a large amount, with the production of these dehydrogenases during the growth and metabolic process. *Hyphomicrobium* belongs to methylotrophic bacteria, which cannot utilize methane but can utilize other one-carbon compounds such as methanol for growth.

In the prior art, various methods have been disclosed to increase the yield of PQQ through the fermentation of *Hyphomicrobium.* In CN106282044A filed in 2015, a strain was obtained after mutagenesis, which has a potency of 1783 mg/L in an 80-m³ fermenter and the potency level is high within the current strains, but when the fermentation volume for the strain was enlarged from a fermenter of 15 m³ to a fermenter of 80 m³, the cumulated methanol feeding was respectively 20% and 27%, and the large difference in methanol consumption between batches is not beneficial to regulating and controlling the fermentation process. CN105624084A filed in 2016 discloses a method for oriented domestication of *Hyphomicrobium denitrificns,* with the potency of the obtained strain being not more than 23 mg/L at best. CN107384984A filed in 2017 discloses a special culture medium, with the potency increasing from 0.57 g/L to 1.3 g/L. CN108949846A filed in 2018 discloses a method for high-density fermentation, with the potency increasing from 0.5-1.0 g/L to 2.0-2.5 g/L. CN110195032A filed in 2019 discloses a strain having a potency of 870 mg/L. CN112375792A filed in 2020 discloses a special fermentation process, with the potency increasing from 320.5 µg/mL to 340.5 µg/mL. CN113699194A filed in 2021 discloses a method for redox potential control, with the potency increasing from 1420.29 mg/L to 2070.41 mg/L. In summary, in the prior art, the fermentation level of *Hyphomicrobium denitrificns* is generally improved by adding a special culture medium or modifying a fermentation method, while a mutagenized strain having a high potency is rarely reported. In addition, PQQ is a byproduct of the metabolic process of *Hyphomicrobium denitrificns,* and the main function thereof is to activate the activity of the methanol dehydrogenase to oxidize methanol into formaldehyde. Though methanol is a carbon source and an energy source for *Hyphomicrobium denitrificns,* its excessive concentration causes damage to the cells or even cell death. Therefore, enhancing the PQQ production ability of *Hyphomicrobium denitrificns* (especially those strains requiring less methanol and having a higher yield) is a major challenge.

Based on the current state of the prior art, besides the need to increase the potency of strains, the main problem to be overcome is how to effectively reduce the cost and conveniently regulate and control fermentation in the scaled-up industrial production.

### SUMMARY

In order to solve the aforementioned problems, the present invention provides a strain taxonomically named as *Hyphomicrobium denitrificns* MQ004-2, which is deposited at the China Center for Type Culture Collection on July 8, 2022, with an accession number of CCTCC NO: M 20221066 and the depository address of Wuhan University, Wuhan, China.

The strain of *Hyphomicrobium denitrificns* provided in the present invention has a PQQ fermentation potency of 1982-2349 µg/mL after 168-200 hours of fermentation in a 50-L fermenter and without using a special method to improve the fermentation level, and a harvest potency of 2137 µg/mL can be achieved after 200 hours of fermentation in a 20-m³ fermenter. The fermentation level of the aforementioned strain is higher than that of the existing strains of *Hyphomicrobium denitrificns,* and the fermentation requires a relatively short time of not more than 200 hours. Therefore, not only the fermentation time of the strain provided in the present invention is short, but also the fermentation level is high.

Methanol is a carbon source necessary for PQQ production using *Hyphomicrobium denitrificns,* while the methanol feeding does not significantly increase the fermentation volume from the early stage to the late stage of the fermentation. In addition, according to the measurement of the applicant, for 10 tons of fermentation broth, the cumulated amount of methanol fed-batch feeding is 20%, which accounts for about 25% of the total production costs. Therefore, on the premise of ensuring the fermentation level, further reduction of methanol consumption is beneficial to reducing the cost. Using the strain provided in the present invention, the consumption of methanol is also effectively reduced, and the maximum cumulated amount of methanol fed-batch feeding is 16% after the fermentation is finished. Based on a calculation, if the fermentation broth had a volume of 10 m³, and the cumulated amount of methanol fed-batch feeding was reduced from 20% to 16%, the methanol consumption would be reduced by 400 L. If the time consumption in fermentation is comprehensively considered, the shorter the fermentation time is, the lower the cost will be, on the premise of obtaining the same potency. Therefore, compared to the prior art, the strain provided in the present application costs less and is more suitable for scaled-up industrial use. Based on the characteristics of the strain and in comparison to the prior art, the strain of *Hyphomicrobium denitrificns* provided in the present invention can be used to achieve a higher potency in a shorter time under the condition of less methanol consumption, i.e., more PQQ is induced using less amount of methanol in a shorter time.

The metabolic process of the strain of *Hyphomicrobium denitrificns* provided in the present invention is stable. For example, for a 50-L fermenter, the cumulated amount of methanol fed-batch feeding is 14-16%, and the difference in methanol consumption among batches is small. After the fermenter is enlarged to 20 m³, the cumulated amount of methanol fed-batch feeding is 15%, the change being insignificant. Because the difference in the methanol consumption among batches is small and the metabolism of the strain is stable, the regulation and control of the fermentation process are finally facilitated, having more advantages in scaled-up industrial production.

The present invention further provides use of the strain of *Hyphomicrobium denitrificns* in the preparation of pyrroloquinoline quinone; or in the preparation of one or more of human medicines, veterinary medicines, health products, foods, drinks, cosmetics, additives, and feed containing pyrroloquinoline quinone.

The present invention further provides a fermentation broth containing the strain of *Hyphomicrobium denitrificns.*

The present invention further provides use of the fermentation broth in the preparation of pyrroloquinoline quinone; or in the preparation of one or more of human medicines, veterinary medicines, health products, foods, drinks, cosmetics, additives, and feed containing pyrroloquinoline quinone.

The present invention further provides a fermentation method for the production of pyrroloquinoline quinone by using the strain of *Hyphomicrobium denitrificns.*

Preferably, the fermentation method comprises performing fermentation in a fermentation medium containing an assimilable carbon source and/or nitrogen source.

Preferably, the assimilable carbon source is methanol, methylamine, or a combination of methanol with other carbon sources, and the other carbon sources are at least selected from one or a combination of any of methylamine, ethanol, glycerol, formic acid, acetic acid, mannitol, starch, maltodextrin, glucose, sucrose, lactose, maltose, industrial molasses, soybean oil, and sorbitol.

Preferably, the assimilable nitrogen source is at least selected from one or a combination of any of yeast extract powder, yeast powder, yeast extract, soybean lecithin, soybean cake powder, cottonseed cake powder, peanut cake powder, gluten powder, corn syrup dry powder, soybean meal, peptone, urea, ammonium salt, and nitrate.

Preferably, the fermentation medium further comprises inorganic salts, wherein the inorganic salts are at least selected from one or a combination of any of trisodium citrate, potassium dihydrogen phosphate, dipotassium hydrogen phosphate, ammonium sulfate, calcium carbonate, ferrous sulfate, zinc sulfate, copper sulfate, sodium chloride, potassium chloride, calcium chloride, magnesium sulfate, ferric chloride, and manganese sulfate.

Preferably, the fermentation process comprises methanol feeding control.

Preferably, after the methanol in the substrate is consumed to below 3 g/L, the fed-batch feeding of methanol is started, and the methanol residue is controlled to be below 2 g/L.

### Beneficial Effects:

1. of the strain provided in the present invention, the fermentation level is significantly higher than the existing levels, and the required fermentation time is short;
2. compared to the prior art, the methanol consumption is further reduced on the premise of a high fermentation level of the strain provided in the present invention; and
3. the fermentation process is easily regulated and controlled, thus large-scale production is easily enabled by using the strain provided in the present invention, as the potency level between batches is stable, the difference in methanol consumption between batches is small, and the metabolism of the strain is stable.

### DETAILED DESCRIPTION

Unless otherwise specified, the experimental methods used in the following examples are all conventional methods.

Unless otherwise specified, the materials, reagents, and the like used in the following examples are all ordinary and commercially available products that can be purchased from a market.

The present invention is to be further described by means of the examples below, and these descriptions are not intended to further limit the content of the present invention. It should be appreciated by those skilled in the art that equivalent substitutions or corresponding modifications made to the content of the present invention still fall within the protection scope of the present invention.

The percentage concentrations of the reagents in the following examples are mass percentages, unless otherwise specified.

### Example 1

### Isolation and screening of strains

Fresh activated sludge was taken from a methanol waste water treatment tank and was centrifuged to remove the supernatant. The resulting activated sludge was washed twice and subsequently resuspended to the original volume with normal saline, and inoculated to an enrichment medium 1 according to the inoculation amount of 1%. Enrichment fermentation broth 1 was obtained from 6 d of cultivation on a shaker. Enrichment fermentation broth 1 was subsequently transferred to an enrichment medium 2 according to the transferring amount of 1%. Enrichment fermentation broth 2 was obtained from 6 d of cultivation on a shaker. Enrichment fermentation broth 2 was subsequently transferred to an enrichment medium 3 according to the transferring amount of 1%. Enrichment fermentation broth 3 was obtained from 6 d of cultivation on a shaker. PQQ content quantitative determination was carried out following each transfer using an HPLC method, and enriched microbial floras were obtained by three times of enrichment culture. Within the enrichment fermentation broths, enrichment fermentation broth 3 had the highest potency.

Enrichment fermentation broth 3 was serially diluted and successively applied onto a screening plate. Single colonies having good growth and full shapes were selected and sequentially inoculated into a seed culture medium and cultured in an incubator at 30-32 °C until the bacterial solution became turbid. Subsequently, the bacterial solution was inoculated into the fermentation medium at the inoculation amount of 0.05 OD and cultured for 8 d. Methanol feeding was carried out at 1.2% on day 3 and the potency was examined once two days using an HPLC method from the start of day 4. MQ-C-03 single colony having the highest potency was selected as the strain to be mutagenized.

The basal formulation of the culture medium was: 0.4% of ammonium sulfate, 0.14% of potassium dihydrogen phosphate, 0.3% of disodium hydrogen phosphate, 0.1% of magnesium sulfate heptahydrate, 0.15% of sodium glutamate, 1 mL/L of mineral water, 1 mL/L of vitamin supplement solution, and a pH of 6.8-7.0.

Preparation of mineral water:
3.75 g of FeSO₄·7H₂O was weighed and dissolved in 10 mL of stock solution A; 6 g of CaCl₂·2H₂O was weighed and dissolved in 50 mL of pure water and mixed with stock solution A; 20 mL of stock solution B was added in subsequently, and the mixture was diluted to 100 mL with pure water in a volumetric flask.
Stock solution A: 22.5 g of ZnSO₄·7H₂O, 3.3 g of MnSO₄·5H₂O, 0.75 g of CuSO₄·5H₂O, and 1.5 g of NaCl were weighed and dissolved in pure water, and the mixture was diluted to 100 mL;
stock solution B: 0.3 g of (NH₄)₆Mo₇O₂₄·4H₂O, 0.3 g of KI, 0.3 g of CoCl₂·6H₂O, and 0.3 g of H₃BO₃ were dissolved in pure water, and the mixture was diluted to 100 mL;
vitamin supplement solution:
2 g of inositol, 8 g of nicotinic acid, 4 g of calcium pantothenate, 2 g of thiamine hydrochloride, 0.05 g of biotin, and 0.02 g of folic acid.

On the basis of the basal formulation, enrichment medium 1 had a methanol addition of 2%, enrichment medium 2 had a methanol addition of 4%, and enrichment medium 3 had a methanol addition of 6%.
Screening plate: basal medium + 2% of agar + 3% of methanol
Seed culture medium: basal medium + 1% of methanol
Fermentation medium: basal medium + 2.5% of methanol + 0.2% of glycerol

### Example 2

### Mutagenesis screening

Strain MQ-C-03 was subjected to mutagenesis treatment using ARTP. Strain MQ-C-03 was cultured in the seed culture medium until OD was approximately 5; 5 mL of bacterial suspension was centrifuged and the supernatant was discarded, the precipitate was washed twice with PBS buffer solution and resuspended. 100 µL of Bacterial suspension was applied onto a sterile screening plate and placed on the carrier of the mutagenesis system for 60 s of irradiation under the configuration of 100 W incident power and 10 SLM helium flow, and the bacterial suspension was eluted with 2 mL of 20% glycerol and mixed uniformly.

NTG was added to the eluted bacterial suspension at an amount of 5 mg/mL. The bacterial suspension was cultured at 30 °C in the dark for 60 min and centrifuged for 10 min; the supernatant was discarded, and the precipitate was washed twice with PBS and resuspended.

The bacterial suspension after NTG mutagenesis was applied onto a screening plate and cultured at 30 °C for 8 d, single colonies having full shapes were selected for potency assay, and strain MQ004-2 with high potency in shake flask fermentation was selected.

The strain was deposited at the China Center for Type Culture Collection on July 8, 2022, with the accession number of CCTCC NO: M 20221066 and the address of Wuhan University.

### Example 3

### Strain identification

The morphologic characteristics of strain MQ004-2: the strain belongs to gram-negative bacteria, colonies of the strain are small and milky white with luster on the surface, and the colonies have a circular shape and a smooth margin; under an optical microscope, the strain is spindle-fusiform and small morphologically, and has filamentous hyphae at two poles.

The physiological and biochemical characteristics of strain MQ004-2: the strain belongs to chemoorganotrophic aerobic bacteria and can utilize methanol, formic acid, acetic acid, arabinose, tagatose, trehalose, and mannitol but not glucose and maltose. The oxidase and catalase contact were positive, and the phosphatase, esterase, methyl red assay, and V-P assay were negative. The strain can reduce nitrates and cannot produce indole and hydrogen sulfide; The strain is sensitive to tetracycline but not to ampicillin, streptomycin, kanamycin, and chloramphenicol.

16srDNA sequencing identification was carried out on strain MQ004-2 and a resultant sequence was obtained. The sequence was subjected to sequence alignment in the NT database of NCBI and was retrieved in the NCBI taxonomy database. Results demonstrated that the strain had the highest homology to *Hyphomicrobium denitrificns* strain ATCC 51888, reaching 99%. Therefore the strain was identified as *Hyphomicrobium denitrificns.*

### Example 4

### Fermentation in a 50-L fermenter

### Shake flask seed

100 µL of glycerol tube seed solution of strain MQ004-2 was inoculated into a seed culture medium shake flask (a 500-mL triangular flask containing 100 mL of the medium) and cultured for 24 h on a shaker in which the temperature was controlled to 30 °C and the rotation was 180 rpm/min. When OD₆₀₀ was detected to be 3.0-5.0, the shake flask seed was inoculated into the seed tank at an inoculation amount of 1.0%.

### Fermentation in a seed tank

10 L of seed culture medium was prepared and loaded into a 20-L seed tank and sterilized at 121 °C for 25 min. After the medium was cooled, the shake flask seed was inoculated therein and cultured with the culture temperature of the seed tank being set to 28-32 °C, the stirring rotation speed being set to 150-400 rpm/min, the level of the dissolved oxygen being ≥ 30% by a stirring linkage, the airflow being 0.5-1.0 VVM, and the culture period being 32-60 h. When OD₆₀₀ was detected to be 3.0-5.0, the seed solution could be transferred to the fermentation culture at the transferring amount of 10%.

### Fermentation culture

30 L of fermentation medium was prepared according to the following formulation and loaded into a 50-L fermenter and sterilized at 121 °C for 25 min. After the medium was cooled, the seed solution was transferred therein and cultured with the culture temperature of the seed tank being set to 28-32 °C, the stirring rotation speed being set to 150-500 rpm/min, the level of the dissolved oxygen being ≥ 10% by a stirring linkage, the airflow being 0.5-1.5 VVM, and the culture period being 168 h.

### Fermentation feeding control

After the methanol in the substrate was consumed to below 3 g/L, methanol fed-batch feeding was started, and the methanol residue was controlled to be below 2 g/L, wherein the method for quickly detecting methanol was to stop methanol feeding, and when the dissolved oxygen did not rise back within 15 min, the methanol accumulation was deemed to exceed 2 g/L, in this case, the amount of the methanol feeding needed to be reduced, and the cumulated amount of methanol fed-batch feeding (the percentage of methanol feeding amount / initial fermentation volume) was 14% in the process.

Dissolved oxygen control in fermentation: the level of the dissolved oxygen in fermentation needed to be controlled to be 30-40% at 0-40 h, 10-20% at 40-80 h, and 20-40% at 80 h-harvest.

OD₆₀₀, pH, methanol content, and potency were detected in the fermentation process, and the harvest potency of the fermentation was 2032 µg/mL.

Culture medium formulation of the seed tank:
2.0% of methanol, 0.4% of ammonium sulfate, 0.14% of potassium dihydrogen phosphate, 0.3% of disodium hydrogen phosphate, 0.1% of magnesium sulfate heptahydrate, 0.15% of sodium glutamate, 0.7 mL/L of mineral water, and a pH of 6.8-7.0.

Fermentation medium formulation:
2.0% of methanol, 0.4% of ammonium sulfate, 0.14% of potassium dihydrogen phosphate, 0.3% of disodium hydrogen phosphate, 0.1% of magnesium sulfate heptahydrate, 0.15% of sodium glutamate, 0.5% of sodium aspartate, 0.7 mL/L of mineral water, 1 mL/L of vitamin supplement solution, and a pH of 6.8-7.0.

### Example 5

### Fermentation in a 50-L fermenter

The procedure was identical to Example 4, which differed in that the cultivation period was changed to 184 h in the fermentation culture. OD₆₀₀, pH, methanol content, and potency were detected in the fermentation process, and results showed that the harvest potency of the fermentation was 1982 µg/mL and the cumulated fed-batch feeding of methanol was 16% in the process.

### Example 6

### Fermentation in a 50-L fermenter

The procedure was identical to Example 4, which differed in that the cultivation period was changed to 200 h in the fermentation culture. OD₆₀₀, pH, methanol content, and potency were detected in the fermentation process, and results showed that the harvest potency of the fermentation was 2349 µg/mL and the cumulated fed-batch feeding of methanol was 16% in the process.

### Example 7

### Fermentation in a 20-m³ fermenter

### Shake flask seed

100 µL of glycerol tube seed solution of strain MQ004-2 was inoculated into a seed culture medium shake flask (a 500-mL triangular flask containing 100 mL of the medium) and cultured for 24 h on a shaker in which the temperature was controlled to 30 °C and the rotation was 180 rpm/min. When OD₆₀₀ was detected to be 3.0-5.0, the shake flask seed was inoculated into the seed tank at an inoculation amount of 1.0%.

### Primary seed tank

A 10-L seed culture medium was prepared and loaded into a 20-L seed tank and sterilized at 121 °C for 25 min. After the medium was cooled, the shake flask seed was inoculated therein and cultured with the culture temperature of the seed tank being set to 28-32 °C, the stirring rotation speed being set to 150-400 rpm/min, the level of the dissolved oxygen being ≥ 30% by a stirring linkage, the airflow being 0.5-1.0 VVM, and the culture period being 32-60 h. When OD₆₀₀ was detected to be 3.0-5.0, the seed solution could be transferred to a secondary seed tank for cultivation at the transferring amount of 1%.

Culture medium formulation of the primary seed tank: 2.0% of methanol, 0.4% of ammonium sulfate, 0.14% of potassium dihydrogen phosphate, 0.3% of disodium hydrogen phosphate, 0.1% of magnesium sulfate heptahydrate, 0.15% of sodium glutamate, and 0.7 mL/L of mineral water, and the pH was controlled to 6.8-7.0 using ammonia solution.

### Secondary seed tank

A 1000-L seed medium was prepared and loaded into a 2000-L fermenter and sterilized at 121 °C for 25 min. The primary seed solution was transferred therein at the transferring amount of 1% and cultured with the culture temperature of the seed tank being set to 28-32 °C, the stirring rotation speed being set to 150-400 rpm/min, the level of the dissolved oxygen being ≥ 30% by a stirring linkage, the airflow being 0.5-1.0 VVM, and the culture period being 32-60 h. When OD₆₀₀ was detected to be 3.0-5.0, the secondary seed solution could be transferred to a fermenter for cultivation at the transferring amount of 10%.

Culture medium formulation of the secondary seed tank: 2.0% of methanol, 0.4% of ammonium sulfate, 0.14% of potassium dihydrogen phosphate, 0.3% of disodium hydrogen phosphate, 0.1% of magnesium sulfate heptahydrate, 0.15% of sodium glutamate, and 0.7 mL/L of mineral water, and the pH was controlled to 6.8-7.0 using ammonia solution.

### Fermentation culture

A 10-m³ fermentation medium was prepared and loaded into a 20-m³ fermenter. In the fermenter, the culture temperature of the seed tank was set to 28-32 °C, the stirring rotation speed was set to 80-150 rpm/min, the level of the dissolved oxygen was ≥ 10% by a stirring linkage, the airflow was 0.5-1.5 VVM, and the culture period was 200 h.

The formulation of the fermentation medium: 2.0% of methanol, 0.4% of ammonium sulfate, 0.14% of potassium dihydrogen phosphate, 0.3% of disodium hydrogen phosphate, 0.1% of magnesium sulfate heptahydrate, 0.15% of sodium glutamate, 0.5% of sodium aspartate, 0.7 mL/L of mineral water, and 1 mL/L of vitamin supplement solution, and the pH was controlled to 6.8-7.0 using ammonia solution.

Fermentation feeding control: after the methanol in the substrate was consumed to below 3 g/L, methanol fed-batch feeding was started, and the methanol residue was controlled to be below 2 g/L, wherein the method for quickly detecting methanol was to stop methanol feeding, and when the level of the dissolved oxygen did not rise back within 15 min, the methanol accumulation was deemed to exceed 2 g/L, and in this case, the feeding amount of the methanol needed to be reduced, and the cumulated amount of methanol fed-batch feeding was 15% in the process.

Dissolved oxygen control in fermentation: the level of the dissolved oxygen in fermentation needed to be controlled to be 30-40% at 0-40 h, 10-20% at 40-80 h, and 20-40% at 80 h-harvest.

OD₆₀₀, pH, methanol content, and potency were detected in the fermentation process, and the harvest potency of the fermentation was 2137 µg/mL.

### Comparative Examples 1-3

The original strain (MQ-C-03 strain before mutagenization) was preserved in glycerol tubes, and the strain was subsequently cultured for 168 h, 184 h, and 200 h respectively according to the operation steps of Example 4, Example 5, and Example 6, and the harvest potency of the fermentation was as follow:

| Comparative Example | Fermentation time (h) | Fermentation potency (µg/mL) | The amount of methanol feeding/initial fermentation volume (%) |
|---|---|---|---|
| 1 | 168 | 1139 | 22 |
| 2 | 184 | 1345 | 19 |
| 3 | 200 | 697 | 17 |

Compared to the original strain, the potency of strain MQ004-2 after mutagenesis was 1982-2349 µg/mL, the potency of the original strain was 697-1345 µg/mL, and the potency level of production in a fermenter of strain MQ004-2 was more stable. In the aspect of methanol consumption, the cumulated amount of methanol fed-batch feeding was 14-16% in strain MQ004-2 and was 17-22% in the original strain. The difference in the methanol consumption among each batch is small in strain MQ004-2, suggesting a stable metabolism of the strain provided in the present application, therefore facilitating the regulation and control of the fermentation process, enabling large-scale production, and reducing methanol consumption significantly.

## Claims

1. A strain of *Hyphomicrobium denitrificns* MQ004-2, deposited at the China Center for Type Culture Collection on July 8, 2022, with an accession number of CCTCC NO: M 20221066.

2. Use of the strain according to claim 1, comprising:
the preparation of pyrroloquinoline quinone; or the preparation of one or more of human medicines, veterinary medicines, health products, foods, drinks, cosmetics, additives, and feed containing pyrroloquinoline quinone.

3. A fermentation broth comprising the strain according to claim 1.

4. Use of the fermentation broth according to claim 3, comprising:
the preparation of pyrroloquinoline quinone; or the preparation of one or more of human medicines, veterinary medicines, health products, foods, drinks, cosmetics, additives, and feed containing pyrroloquinoline quinone.

5. A fermentation method for the production of pyrroloquinoline quinone by using the strain according to claim 1.

6. The fermentation method according to claim 5, comprising:
performing fermentation in a fermentation medium containing an assimilable carbon source and/or nitrogen source.

7. The fermentation method according to claim 6, comprising:
the assimilable carbon source is methanol, methylamine, or a combination of methanol with other carbon sources, and the other carbon sources are at least selected from one or a combination of any of methylamine, ethanol, glycerol, formic acid, acetic acid, mannitol, starch, maltodextrin, glucose, sucrose, lactose, maltose, industrial molasses, soybean oil, and sorbitol.

8. The fermentation method according to claim 6, comprising:
the assimilable nitrogen source is at least selected from one or a combination of any of yeast extract powder, yeast powder, yeast extract, soybean lecithin, soybean cake powder, cottonseed cake powder, peanut cake powder, gluten powder, corn syrup dry powder, soybean meal, peptone, urea, ammonium salt, and nitrate.

9. The fermentation method according to claim 6, comprising:
the fermentation medium further comprises inorganic salts, wherein the inorganic salts are at least selected from one or a combination of any of trisodium citrate, potassium dihydrogen phosphate, dipotassium hydrogen phosphate, ammonium sulfate, calcium carbonate, ferrous sulfate, zinc sulfate, copper sulfate, sodium chloride, potassium chloride, calcium chloride, magnesium sulfate, ferric chloride, and manganese sulfate.

10. The fermentation method according to any one of claims 5-9, wherein
the fermentation process comprises methanol feeding control.
